# EUROPEAN PATENT APPLICATION

(11) **EP 4 847 075 A1**
(43) Date of publication of application: **02.12.2026**
(21) Application number: 26174750.5
(22) Date of filing: 27.04.2026
(51) Int. Cl.: A61M 39/28, F16K 7/04, F16K 7/06

(54) **PINCH VALVE**

(30) Priority: 29.05.2025 US 202563813737 P; 11.11.2025 US 202519385626
(71) Applicant: SMC Corporation, Tokyo 104-0031 (JP)
(72) Inventor: YAMANASHI, Masanobu, Chiba, 277-0871 (JP); TATE, Bianca, Noblesville, Indiana 46060 (US)
(74) Representative: Keil & Schaafhausen Patentanwälte PartGmbB

(57) **Abstract**

[Object] To provide a pinch valve excellent in space efficiency in addition to operability.

[Solution] A pinch valve 100 includes: a holder 10 having a holding groove 12 for holding a tube 80 made of an elastic material; a press portion 50 that is supported so as to be able to reciprocate in a direction along a first axis L1 in the holding groove and that is configured to squeeze the tube held in the holding groove; and a drive unit 70 configured to reciprocate the press portion. The holding groove extends in a direction along a second axis L2 orthogonal to the first axis and has an opening 17 that is open along the first axis at one end in the direction along the first axis and through which the tube can be inserted into and removed from the holding groove, and the holder includes a cover arm 2 configured to open and close the opening.

## Description

### Technical Field

The present invention relates to a pinch valve configured to block or control a medium such as a liquid flowing in a soft tube.

### Background Art

Pinch valves configured to squeeze a tube in which a liquid such as a liquid medicine flows and that is made of an elastic material to control the flow state of the liquid in the tube have been publicly known as disclosed in, for example, PTLs 1 and 2. The pinch valves disclosed in PTLs above are capable of controlling the flow of a liquid, for example, so as to block the flow of the liquid by squeezing a tube by a press lever advanced in a state in which the tube is inserted into and held by a holding groove that is open in a holder.

However, in each of the pinch valves disclosed in PTLs above, an opening through which the tube is inserted into the holding groove faces in a direction orthogonal to the direction in which the press lever advances or retreats in the holder, that is, toward sides of the pinch valve. Thus, to insert or remove the tube into or from the holding groove, the tube has to be inserted into or removed from the holding groove from a side of the holder, and this operation is likely to be complicated. In addition, a space for allowing the tube to access the opening of the holding groove has to be secured on the side of the holder. Thus, more efficiently mountable pinch valves are required to be developed.

### Citation List

### Patent Literature

PTL 1: Japanese Unexamined Patent Application Publication No. 2004-324730
PTL 2: Japanese Unexamined Patent Application Publication No. 2023-160330

### Summary of the Invention

Accordingly, a technical object of the present invention is to provide a pinch valve excellent in space efficiency in addition to operability.

### Solution to Problem

To solve the above problem, the present invention provides a pinch valve including: a holder having a holding groove for holding a tube made of an elastic material; a press portion that is supported so as to be able to reciprocate in a direction along a first axis in the holding groove and that is configured to squeeze the tube held in the holding groove; and a drive unit configured to reciprocate the press portion, in which the holding groove extends in a direction along a second axis orthogonal to the first axis and has an opening that is open along the first axis at one end in the direction along the first axis and through which the tube can be inserted into and removed from the holding groove, and the holder includes a cover arm configured to open and close the opening.

In the present invention, preferably, the holder includes a first frame and a second frame that extend along the first axis with the first axis interposed therebetween, the holding groove being formed between a pair of the frames, and the first frame has a pivot axis parallel to the first axis, the cover arm being supported so as to be pivotable on the first frame around the pivot axis between a closed position in which the cover arm closes the opening and an open position in which the cover arm opens the opening.

In addition, preferably, the cover arm in the closed position is detachably locked into the second frame in a pivoting direction around the pivot axis.

In addition, preferably, the cover arm includes a lock pin provided so as to be able to advance and retreat, the second frame having an engagement recess with which the advanced lock pin is engaged, the lock pin and the engagement recess being configured to be engageable with each other in the closed position.

In addition, preferably, the lock pin is provided so as to be able to advance toward a tip end side of the cover arm and retreat and is always urged by an urging spring in an advance direction of the lock pin.

In addition, preferably, a tip end portion of the lock pin has a follow inclined surface, the lock pin being configured to retreat against an urging force of the urging spring by the follow inclined surface coming into contact with the second frame when the cover arm pivots to the closed position.

In addition, preferably, the cover arm includes an operation lever for releasing engagement between the lock pin and the engagement recess by retreating the lock pin against the urging force of the urging spring.

In addition, preferably, in the present invention, a tip end portion of the cover arm has a contact surface formed so as to face one end side in the direction along the first axis, the second frame having a stopper surface formed so as to face another end side in the direction along the first axis, and in the closed position of the cover arm, the contact surface and the stopper surface are disposed so as to face each other. In addition, preferably, the first frame further has a stopper surface formed so as to face the other end side in the direction along the first axis.

In addition, in the present invention, the holding groove may further have, at respective end portions thereof in the direction along the second axis, arc-shaped support surfaces for coming into contact with and supporting an outer peripheral surface of the tube.

In addition, the drive unit may be an air cylinder joined to the other end side of the holder, the air cylinder including a hollow cylinder main body, and a piston configured to reciprocate in the direction along the first axis in the cylinder main body, and the press portion may be connected to the one end side of the piston. In addition, more preferably, the air cylinder includes a press spring configured to always urge the piston toward one of the one end side and the other end side in the direction along the first axis.

### Advantageous Effects of Invention

As described above, in the pinch valve according to the present invention, the opening through which the tube is inserted into and removed from the holder faces in the direction in which the press portion advances or retreats, that is, the front side of the pinch valve. Thus, the tube can be inserted into and removed from the holder by front access. That is, the tube can be easily inserted into and removed from the holding groove from the front side of the holder. In addition, the pinch valve according to the present invention eliminates the need for securing, on the side of the holder, a space for allowing the tube to access the opening compared with the existing pinch valves in which the opening of the holder is located at the side.

As a result, the pinch valve according to the present invention is capable of improving the operability of tube insertion and removal and is capable of improving the space efficiency.

### Brief Description of the Drawings

[Fig. 1] Fig. 1 is an overall perspective view of a pinch valve (in a closed position) according to the present invention.
[Fig. 2] Fig. 2 is an overall exploded perspective view illustrating a main part of the pinch valve (in an open position) according to the present invention.
[Fig. 3(A)] Fig. 3(A) is a diagram illustrating the configuration of a holder main body according to the present invention.
[Fig. 3(B)] Fig. 3(B) is an exploded view of part of the holder main body according to the present invention.
[Fig. 4] Fig. 4 includes perspective views illustrating the configuration of a cover arm according to the present invention.
[Fig. 5] Fig. 5 is a diagram illustrating the configuration of a first embodiment of a drive unit according to the present invention.
[Fig. 6] Fig. 6 is a diagram illustrating the configuration of a second embodiment of the drive unit according to the present invention.
[Fig. 7] Fig. 7 is a diagram illustrating a mounting example of the pinch valve according to the present invention.
[Fig. 8] Fig. 8 includes sectional views of the main part of the pinch valve according to the present invention when the pinch valve is open and closed.

### Detailed Description of the Invention

An embodiment of a pinch valve according to the present invention will be described in detail below with reference to the drawings.

In the drawings, the same components have the same reference signs, and redundant descriptions thereof are omitted. In addition, in the following description, terms such as up, down, left, and right are used on the basis of positions and directions in the drawings and do not limit at all, for example, positions and postures in use.

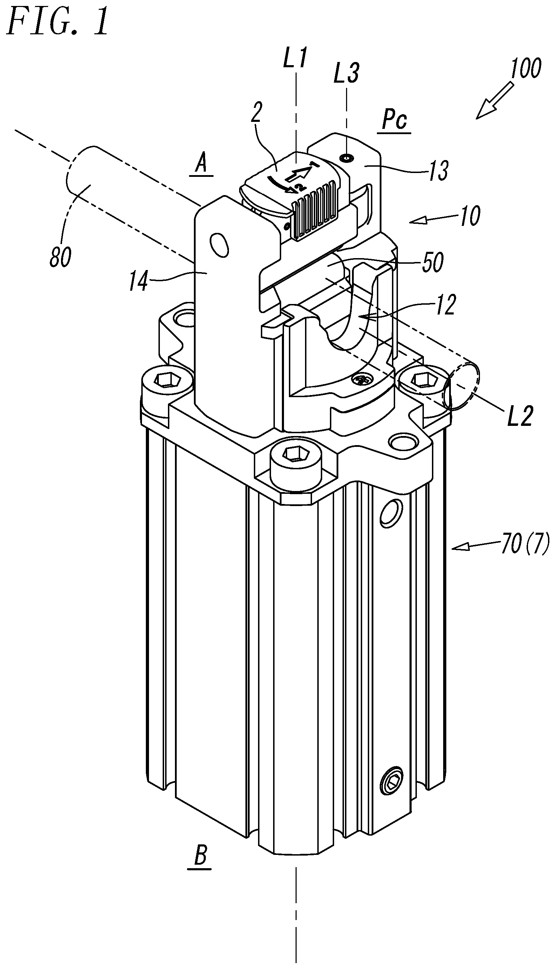

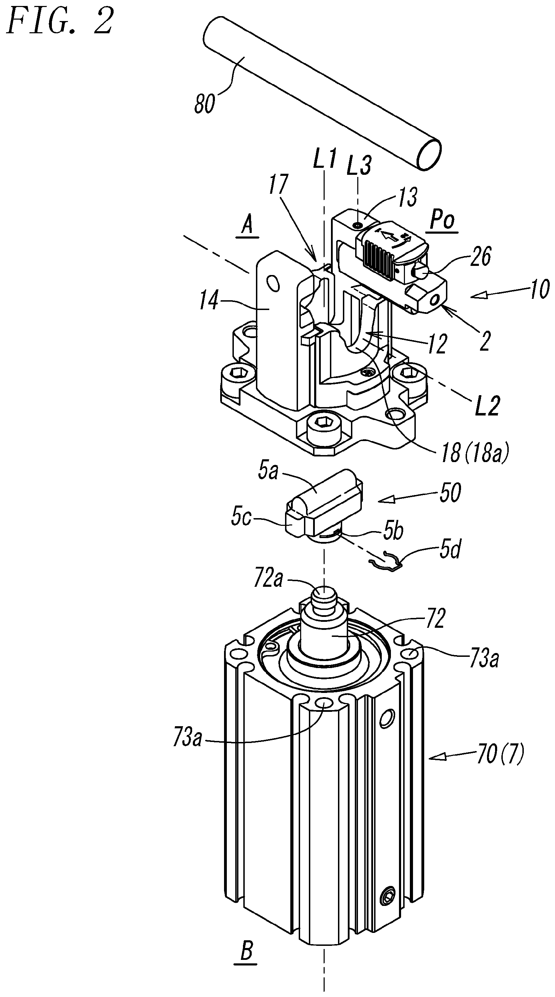

The overall configuration of a pinch valve 100 according to the present invention will be described with reference to Figs. 1 and 2.

Fig. 1 illustrates the pinch valve 100 including a cover arm 2 in a closed position Pc. The pinch valve 100 includes a holder 10 having a holding groove 12 for holding a tube 80 made of an elastic material, a press portion 50 supported so as to be able to reciprocate in a direction along a first axis L1 in the holding groove 12 and configured to squeeze the tube 80 held in the holding groove 12, and a drive unit 70 configured to reciprocate the press portion 50. In the present embodiment, the drive unit 70 is an air cylinder 7. In addition, the holding groove 12 of the holder 10 extends in a direction along a second axis L2 orthogonal to the first axis L1. Here, the pinch valve 100 has, in the direction along the first axis L1, one end A at the tip end of the holder 10, and the other end B at the base end of the drive unit 70. The holder 10 is connected to the one end A side in the direction along the first axis L1 of the drive unit 70.

Fig. 2 illustrates the pinch valve 100 including the cover arm 2 in an open position Po. The holding groove 12 has, at the one end A in the direction along the first axis L1, an opening 17, which is open along the first axis L1 and through which the tube 80 can be inserted into and removed from the holding groove 12. Then, the cover arm 2 opens and closes the opening 17. This configuration of the pinch valve 100 according to the present invention enables the tube 80 to be inserted into and removed from the holding groove 12 in a direction from the one end A side along the first axis L1, along which the press portion 50 reciprocates.

In addition, the holder 10 includes a first frame 13 and a second frame 14, which extend along the first axis L1 with the first axis L1 interposed therebetween. Then, the holding groove 12 is formed between the pair of frames.

In addition, the first frame 13 has a pivot axis L3 parallel to the first axis L1. The cover arm 2 is supported so as to be pivotable on the first frame 13 around the pivot axis L3 between the closed position Pc (Fig. 1) in which the cover arm 2 closes the opening 17 and the open position Po (Fig. 2) in which the cover arm 2 opens the opening 17. Then, the cover arm 2 in the closed position Pc is detachably locked into the second frame 14 in the pivoting direction around the pivot axis L3.

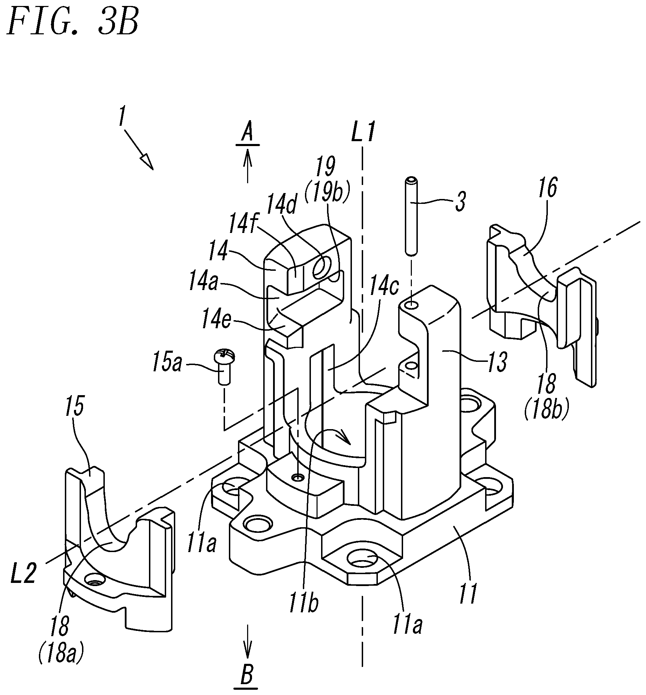

In addition, the cover arm 2 includes a lock pin 26 provided so as to be able to advance and retreat. Then, the second frame 14 has an engagement recess 14d (Fig. 3(B)), with which the advanced lock pin 26 is engaged. With this configuration, the lock pin 26 is locked into the engagement recess 14d in the closed position Pc.

### (Press Portion 50)

In addition, as illustrated in Fig. 2, the press portion 50 has a longitudinal direction along the direction in which the first frame 13 and the second frame 14 face each other and has slide projections 5c extending from respective end faces in the longitudinal direction. In addition, a press member 5a configured to come into contact with and press the tube 80 is formed on the one end A side of the press portion 50. In addition, a connection recess 5b configured to be connected to a piston rod 72 of the drive unit 70 is provided on the other end B side of the press portion 50. The press portion 50 is connected to a connection projection 72a of the piston rod 72 by a fixing clip 5d.

### (Holder 10)

The holder 10 includes a holder main body 1 and the cover arm 2.

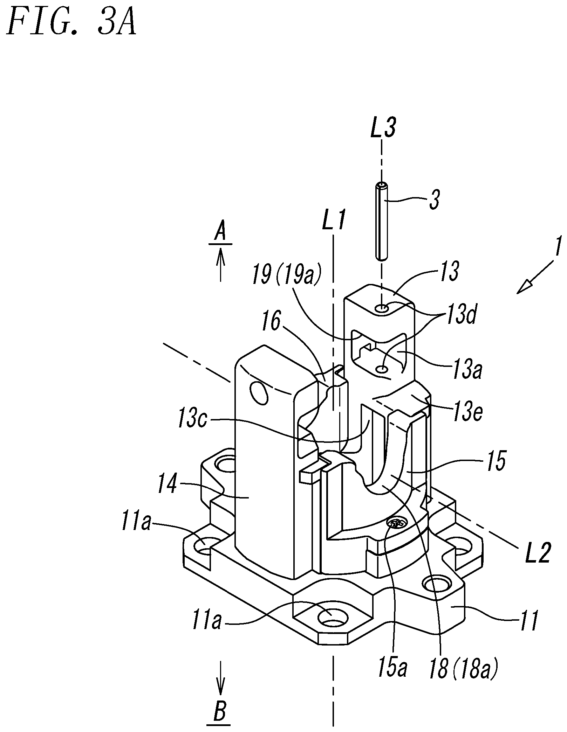

The specific structure of the holder main body 1 will be described with reference to Figs. 3(A) and 3(B).

The holder main body 1 includes, on the other end B side thereof, a holder base 11 including connection portions 11a configured to be connected to the drive unit 70.

The first frame 13 and the second frame 14, which extend parallel to the first axis L1 with the first axis L1 interposed therebetween, stand on a surface of the holder base 11 on the one end A side. A surface of the first frame 13 and a surface of the second frame 14 facing each other are formed parallel to each other and respectively have a first slide groove 13c and a second slide groove 14c, which extend in the direction along the first axis L1 and in which the press portion 50 slides so as to be able to reciprocate.

With this configuration, the press portion 50 smoothly reciprocates in the direction along the first axis L1 such that the slide projections 5c and the first and second slide grooves 13c and 14c of the first and second frames 13 and 14 to be described later are engaged with each other.

### (Holder Base 11)

In addition, a first holding member 15 and a second holding member 16, which form the holding groove 12 extending in the direction along the second axis L2 orthogonal to the first axis L1, stand on the surface of the holder base 11 on the one end A side.

The holding groove 12 has, at respective end portions thereof in the direction along the second axis L2, arc-shaped support surfaces 18 for coming into contact with and supporting an outer peripheral surface of the tube 80. In the present embodiment, as illustrated in Fig. 3, the support surfaces 18 are formed by a first support surface 18a and a second support surface 18b.

In addition, the first holding member 15 and the second holding member 16 are detachably fixed to the holder base 11 with first and second fixing screws 15a and 16a, respectively and can be replaced according to the diameter of the tube 80.

With this configuration, the holding groove 12 has the opening 17, which is formed by the pair of frames (the first frame 13 and the second frame 14) and a pair of holding members (a first holding member 15 and a second holding member 16) and which is open along the first axis L1 at the one end in the direction along the first axis L1. Then, the first holding member 15 and the second holding member 16 form the U-shaped bottom of the holding groove 12. The holding groove 12 extends in the direction along the second axis L2 orthogonal to the first axis L1 and has the opening 17, which is open along the first axis at the one end in the direction along the first axis L1 and through which the tube 80 can be inserted into and removed from the holding groove 12.

In addition, in Fig. 3(B), an internal space surrounded by the first and second frames 13 and 14 and the first and second holding members 15 and 16 is formed. The holder base 11 has, at a position corresponding to the internal space, a through hole 11b passing through the holder base 11 in the direction along the first axis L1. Then, the press portion 50 is disposed so as to be able to reciprocate in the direction along the first axis L1 in the internal space through the through hole 11b.

In the present embodiment, the opening 17 of the holder 10 is a space between the first frame 13 and the second frame 14 facing each other and a part that is open on the one end A side. The opening 17 is opened and closed by the cover arm 2. Then, the cover arm 2 is supported by the first frame 13 so as to be pivotable around the pivot axis L3 and is detachably locked into the second frame 14 in the pivoting direction in the closed position Pc.

The specific configuration of the first frame 13 and the second frame 14 will be described with reference to Figs. 3(A) and 3(B).

In Fig. 3(A), the first frame 13 includes a step portion 13e, in which the height of the first frame 13 in the direction along the first axis L1 is reduced at a position in the width direction of the first frame 13 in the direction along the second axis L2. As a result, the width of the first frame 13 in the direction along the second axis L2 is smaller in the one end A side (high) part than in the step portion 13e. The step portion 13e is formed such that the cover arm 2 does not interfere with the first frame 13 when pivoting from the closed position Pc (Fig. 1) to the open position Po (Fig. 2).

In addition, the first frame 13 has a first recess 13a, which has an inner surface (upper inner surface) on the one end A side in the direction along the first axis L1, and an inner surface (lower inner surface) facing the upper inner surface and which has an opening continuous with a surface on the side of the direction along the second axis L2 in which the cover arm 2 pivots, and a surface facing the second frame 14.

Then, an end face of the first frame 13 on the one end A side has a pivot axis hole 13d passing through the first recess 13a along the pivot axis L3 parallel to the first axis L1.

In addition, a first stopper surface 19a configured to be subjected to a pressing force when the valve is closed is formed at the inner surface (upper inner surface) in the direction along the first axis L1 of the first recess 13a.

In Fig. 3(B), the second frame 14 has a second recess 14a, which has an inner surface (upper inner surface) on the one end A side in the direction along the first axis L1, and an inner surface (lower inner surface) facing the upper inner surface and which has an opening continuous with a surface on the side of the same direction as that of the opening of the first recess 13a in the direction along the second axis L2, and a surface facing the first frame 13. An arm guide inclined surface 14e is formed at the edge of the opening of the second recess 14a in the direction along the second axis L2 such that a tip end portion 21g of the cover arm 2 can smoothly enter the opening in the direction along the second axis L2.

In addition, a surface of the second frame 14 that faces the first frame 13 and is closer to the one end A side (upper side) than the second recess 14a has the engagement recess 14d, with which the lock pin 26 of the cover arm 2 is engaged.

In addition, a lock guide inclined surface 14f that a lock follow inclined surface 26a of the lock pin 26 follows to retreat the lock pin 26 is formed at a surface of the second frame 14 that faces the first frame and is closer to the one end A side (upper side) than the second recess 14a. With this configuration, in a process in which the cover arm 2 moves from the open position to the closed position, the lock pin 26 retreats at the lock guide inclined surface 14f. Then, the lock pin 26 is engaged with the engagement recess 14d and is thus held in the closed position.

In addition, a second stopper surface 19b configured to be subjected to a pressing force when the valve is closed is formed at the inner surface (upper inner surface) on the one end A side in the direction along the first axis L1 of the second recess 14a.

### (Cover Arm 2)

The cover arm 2 opens and closes the opening 17, which is open along the first axis L1. As illustrated in Figs. 1 and 2, the cover arm 2 is supported so as to be pivotable around the pivot axis L3 of the first frame 13 and has a longitudinal direction extending in a radial direction. Then, the cover arm 2 pivots around the pivot axis L3 between the closed position Pc (Fig. 1) in which the cover arm 2 closes the opening 17 and the open position Po (Fig. 2) in which the cover arm 2 opens the opening 17. The cover arm 2 in the open position Po in Fig. 2, that is, the cover arm 2 disposed along the direction along the second axis L2, will be described below.

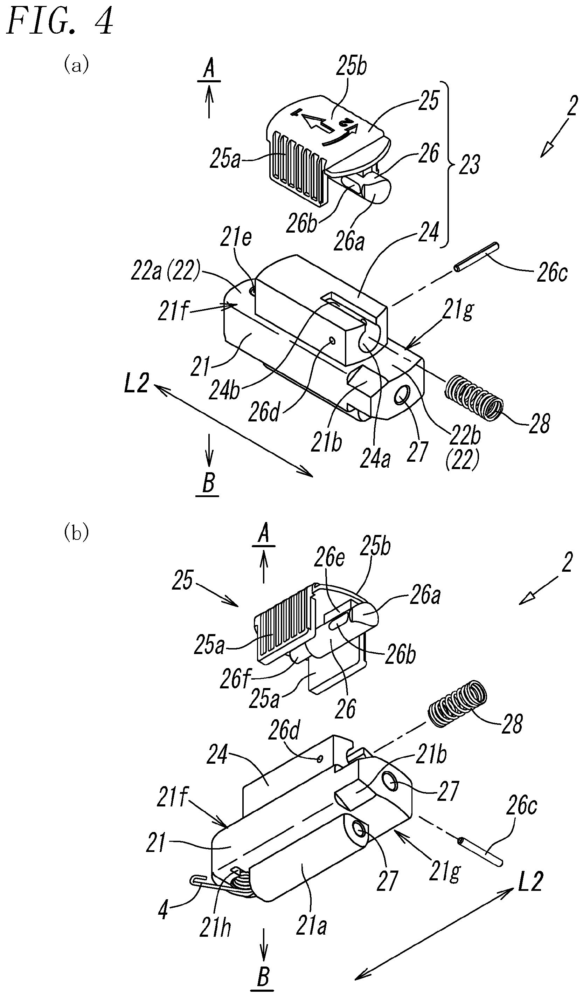

The specific configuration of the cover arm 2 will be described with reference to Fig. 4.

The cover arm 2 includes an arm main body 21, and a lock mechanism 23 on an upper surface of the arm main body 21 on the one end A side.

### (Arm Main Body 21)

In Fig. 4(a), the arm main body 21 has a longitudinal direction along the direction along the second axis L2. The length of the arm main body 21 in the longitudinal direction is set to be larger than the distance between the first frame 13 and the second frame 14 in the opening 17. Then, the arm main body 21 has a base end portion 21f, at one end thereof, supported by the first frame 13 so as to be pivotable, and the tip end portion 21g, at the other end thereof, configured to be locked into the second frame 14. The base end portion 21f has a pivot axis hole 21e in the direction along the pivot axis L3. An end face of the base end portion 21f has an arc shape so as not to interfere with the inside of the first recess during pivoting.

In addition, a first contact surface 22a configured to come into contact with the first stopper surface 19a of the first frame 13 is formed at a surface of the base end portion 21f on the one end A side.

In addition, as illustrated in Fig. 4(b), a torsion spring receiving portion 21h having a large diameter and concentric with the pivot axis hole 21e is formed in a surface of the base end portion 21f on the other end B side. A torsion spring 4 is disposed in the torsion spring receiving portion 21h.

With this configuration, the base end portion 21f is supported by a pivot axis 3 so as to be pivotable in a first recess 13a of the first frame 13. In addition, the torsion spring 4 always urges the cover arm 2 so as to pivot toward the open position Po.

Next, parts of the tip end portion 21g of the arm main body 21 on the side of the pivoting direction toward the closed position have arm follow inclined surfaces 21b. The arm follow inclined surfaces 21b are formed at an upper surface and a lower surface of the arm main body 21 such that the thickness of the arm main body 21 is reduced toward a side surface of the arm main body 21.

With this configuration, when the cover arm 2 pivots to the closed position Pc, the arm follow inclined surface 21b is guided by the arm guide inclined surface 14e of the second frame 14. As a result, the tip end portion 21g of the arm main body 21 can smoothly enter the second recess 14a.

In addition, a second contact surface 22b configured to come into contact with the second stopper surface 19b of the second frame 14 is formed at a surface of the tip end portion 21g on the one end A side.

In addition, a compression member 21a extending in the direction along the second axis L2 between the base end portion 21f and the tip end portion 21g is formed on a surface of the arm main body 21 on the other end B side.

With this configuration, in the closed position Pc, the compression member 21a, together with the first holding portion 15 and the second holding portion 16, can hold the tube 80. In addition, when the valve is closed, the tube 80 is squeezed between the compression member 21a and the press member 5a of the press portion 50. A hole at an end portion of the arm main body 21 in the longitudinal direction is a lightening hole 27 when metal injection molding is performed.

### (Lock Mechanism 23)

Next, the lock mechanism 23 will be described.

As illustrated in Figs. 4(a) and 4(b), the lock mechanism 23 is disposed, between the base end portion 21f and the tip end portion 21g, on the surface of the arm main body 21 on the one end A side. In addition, the lock mechanism 23 is formed by a lock base 24, an operation lever 25 including the lock pin 26, an urging spring 28, and a fixing pin 26c.

The lock base 24 is disposed on the surface (upper surface) of the arm main body 21 on the one end A side so as to have a substantially cuboid shape and have a longitudinal direction along the direction along the second axis L2. A bottomed slide hole 24a, which extends from an end face of the lock base 24 on the tip end portion 21g side toward an end face of the lock base 24 on the base end portion 21f side and whose section has a circular shape, is drilled in the lock base 24. In addition, the lock base 24 has a slit 24b, which passes from a surface (upper surface) thereof on the one end A side into the slide hole 24a and which extends from the end face on the tip end portion 21g side along the slide hole 24a.

The operation lever 25 includes a display board 25b covering the upper surface of the lock base 24. Uneven nonslip grip portions 25a extend, along side surfaces of the lock base 24, from two sides of the display board 25b parallel to the second axis L2 in the open position Po. With this configuration, the operation lever 25 is provided so as to cover part of the upper surface of the lock base 24 and part of each of both the side surfaces of the lock base 24 and can slide relative to the lock base in the direction along the second axis L2. The display board 25b displays the operating procedure for moving the cover arm 2 from the closed position Pc to the open position Po.

In addition, as illustrated in Fig. 4(b), the lock pin 26 having a substantially cylindrical shape is disposed, on a surface (back surface) of the display board 25b on the other end B side, along the direction along the second axis L2 between the grip portions 25a. Then, the display board 25b and the lock pin 26 are connected by a connection wall 26e formed along the direction along the second axis L2.

In addition, an end portion of the lock pin 26 on the tip end portion 21g side has the lock follow inclined surface 26a formed from an end face of the tip end portion to a peripheral surface of the lock pin 26 in the pivoting direction toward the closed position. The lock follow inclined surface 26a (follow inclined surface) in the present embodiment is formed by a curved surface whose outside projects. In addition, an urging spring receiving portion 26f having a small-diameter portion and a step portion is formed at an end portion of the lock pin 26 on the base end portion 21f side.

In addition, the lock pin 26 has, in a peripheral surface closer to the urging spring receiving portion 26f than the lock follow inclined surface 26a, an elongated hole 26b extending in the longitudinal direction and in the direction in which the grip portions 25a face each other.

Here, the coil-shaped urging spring 28 is first inserted into the slide hole 24a of the lock base 24. Next, the lock pin 26 is inserted into the slide hole 24a in a direction from the urging spring receiving portion 26f to the slide hole 24a. Simultaneously, the connection wall 26e is slidably engaged with the slit 24b of the lock base 24. Then, the lock pin 26 is connected to the lock base 24 by the fixing pin 26c in a state in which the urging spring 28 is compressed such that the fixing pin 26c passes through the elongated hole 26b and fixing holes 26d drilled in the side surfaces of the lock base 24. As a result, the lock pin 26 is provided so as to be able to advance toward the tip end side of the cover arm 2 and retreat and is always urged by the urging spring 28 in the advance direction.

With this configuration, the tip end portion of the lock pin 26 has the follow inclined surface 26a, and the follow inclined surface 26a comes into contact with the second frame 14 when the cover arm 2 pivots to the closed position Pc. Thus, the lock pin 26 is configured to retreat against the urging force of the urging spring 28.

In addition, with this configuration, the operation lever 25 retreats the lock pin 26 against the urging force of the urging spring 28 and thus can release the engagement between the lock pin 26 and the engagement recess 14d.

In the present embodiment, the operation lever 25 and the lock pin 26 are formed integrally with each other but may be formed separately from each other.

### (Drive Unit 70)

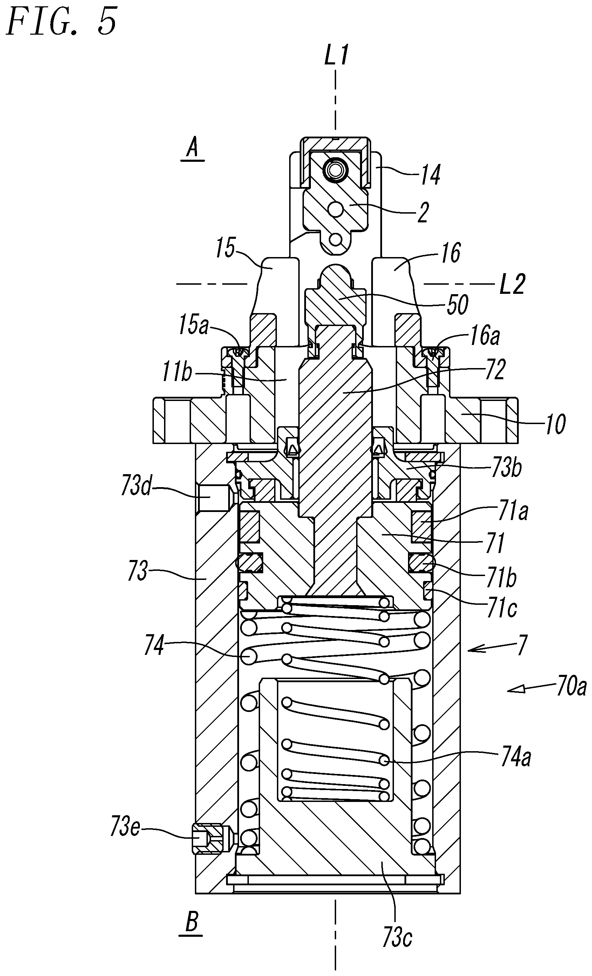

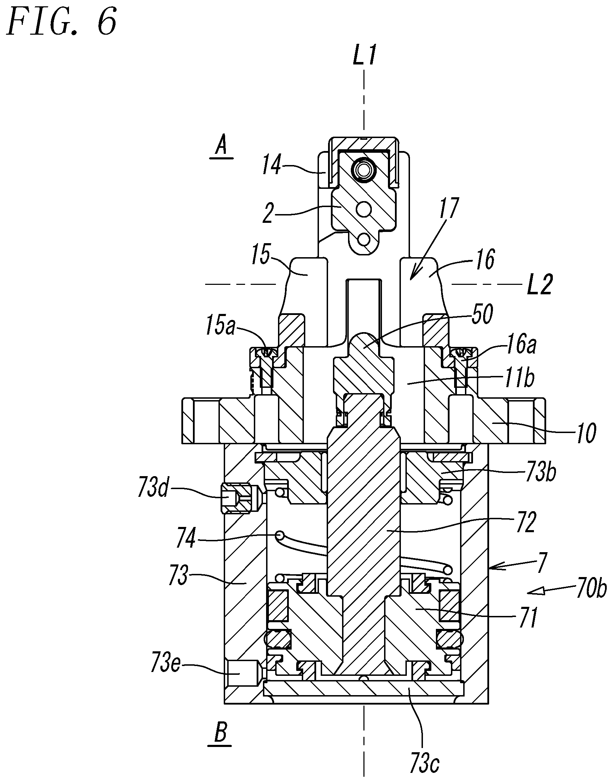

Figs. 5 and 6 illustrate two embodiments (70a and 70b) of the drive unit 70.

In the present embodiment, the drive unit 70 is the air cylinder 7 joined to the other end B side of the holder 10. The air cylinder 7 includes a hollow cylinder main body 73, and a piston 71 configured to reciprocate in the direction along the first axis L1 in the cylinder main body 73. In addition, the press portion 50 is connected to the one end A side of the piston 71. In addition, the air cylinder 7 includes a press spring 74 configured to always urge the piston 71 toward one of the one end A side and the other end B side in the direction along the first axis L1.

A drive unit 70a in Fig. 5 is an air cylinder 7 joined to the other end B side of the holder 10. The air cylinder 7 includes a hollow cylinder main body 73, and a piston 71 configured to reciprocate in the direction along the first axis L1 in the cylinder main body 73. The press portion 50 is connected to the one end A side of a piston rod 72 connected to the piston 71. A detection magnet 71a for position detection, a gasket 71b, and a piston ring 71c are disposed on a slide surface of the piston 71.

In addition, the air cylinder 7 includes press springs 74 configured to always urge the piston 71 toward the one end A side in the direction along the first axis L1. The press springs 74 are disposed between the piston 71 and two spring receiving portions formed to an end-side partition wall 73c closing the other end B side of the air cylinder 7. In the present embodiment, the press springs 74 include an auxiliary press spring 74a.

The pinch valve 100 in the present embodiment is a so-called normally closed valve. Here, air is supplied from a first air inlet and outlet hole 73d communicating with the one end side of the piston 71 of the air cylinder 7 and is discharged from a second air inlet and outlet hole 73e communicating with the other end B side of the piston 71 of the air cylinder 7. As a result, the piston 71 moves toward the other end B side (lower side), and the press portion 50 descends, thus enabling the pinch valve to be in the open state.

A drive unit 70b in Fig. 6 includes a press spring 74 configured to always urge the piston 71 toward the other end B side in the direction along the first axis L1. The press spring 74 is disposed between the piston 71 and a spring receiving portion formed to a rod-side partition wall 73b of the air cylinder 7.

The pinch valve 100 in the present embodiment is a so-called normally open valve. Here, air is supplied from the second air inlet and outlet hole 73e communicating with a pressure chamber on the other end B side of the piston 71 and is discharged from the first air inlet and outlet hole 73d communicating with a pressure chamber on the one end A side of the piston 71. As a result, the piston 71 moves toward the one end side (upper side), and the press portion 50 ascends to squeeze the tube 80, thus enabling the pinch valve to be in the closed state.

In the present embodiment, the air cylinder is a single-acting cylinder but may be a double-acting cylinder. In addition, the drive unit 70 is not limited to such an air cylinder, and it is sufficient that the drive unit 70 be capable of reciprocating the press portion 50. The drive unit 70 may be any of various actuators such as a fluid drive bellows, an electromagnetic actuator, an electric actuator, and a piezoelectric element. In addition, the drive unit 70 is capable of performing control so as to adjust the opening degree of the pinch valve continuously between the open position and the closed position.

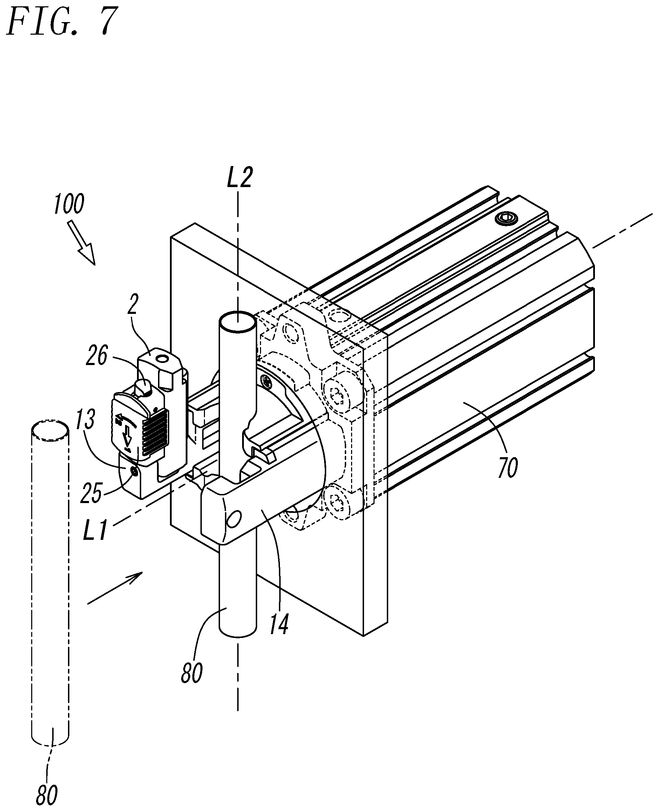

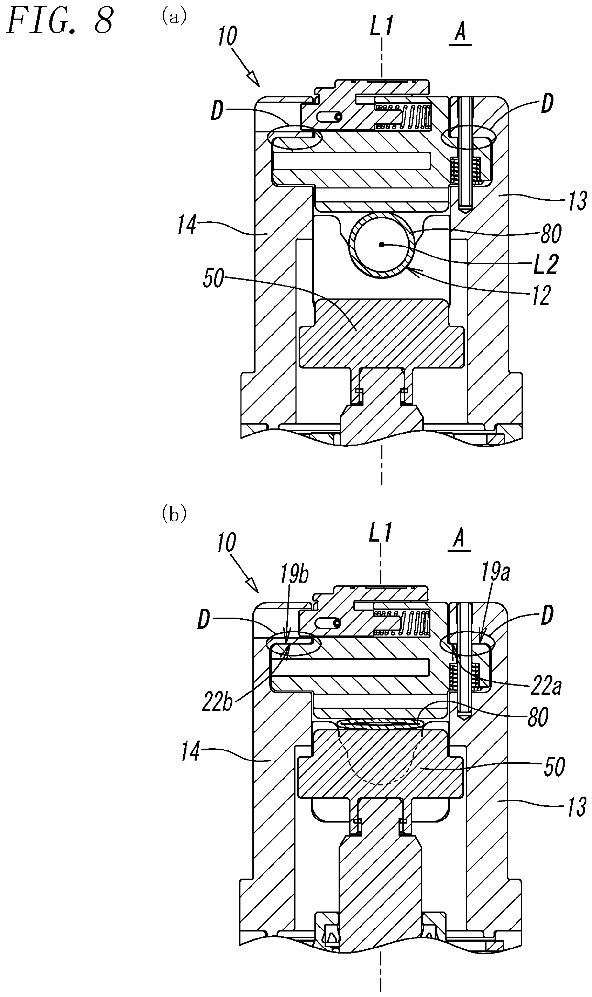

A specific mounting example and a specific operating procedure of the pinch valve 100 according to the present invention will be described with reference to Figs. 7 and 8.

Fig. 7 illustrates a mounting example of the pinch valve 100 according to the present invention.

In the mounting example, the pinch valve 100 is mounted to a mounting wall in a posture in which the cover arm 2 pivots upward such that the first axis L1 is set to be horizontal and that the second axis L2 is set to be along an up-down direction.

The tube 80 can be inserted into and removed from the opening from the front side (front access). The present invention is capable of improving the operability when the tube 80 is attached or detached. In addition, if a plurality of the pinch valves 100 are arranged continuously in a left-right direction, it is possible to make a space between the pinch valves 100 minimum and to thus also improve the space efficiency.

The mounting example is merely an example and is not intended to limit the position and posture in which the pinch valve is mounted. It is possible to mount the pinch valve according to the present invention in various positions and postures.

The specific operating procedure of the pinch valve 100 will be described below.

### (Attachment of Tube 80)

In Fig. 7, the tube 80 in a posture along the direction along the second axis L2 is inserted into the holding groove 12 through the opening 17 in the direction along the first axis L1.

Then, the cover arm 2 pivots in the closing direction against the elastic force of the torsion spring 4. Then, the arm follow inclined surface 21b of the tip end portion 21g of the arm main body 21 follows the arm guide inclined surface 14e formed at the second frame 14, and the tip end portion 21g of the arm main body 21 is thus guided to the second recess 14a. In addition, in conjunction with the pivoting of the cover arm 2, the lock follow inclined surface 26a of the lock pin 26 of the lock mechanism 23 follows the lock guide inclined surface 14f formed at the second frame 14, and the lock pin 26 of the lock mechanism 23 thus moves to the engagement recess 14d against the urging spring 28. The lock pin 26 reaches the engagement recess 14d, is then advanced into the engagement recess 14d by the urging spring 28, and is thus engaged with the engagement recess 14d. By using this procedure, the tube 80 is attached to the pinch valve 100.

### (Holding of Tube 80)

Fig. 8(a) illustrates a state in which the tube 80 is held.

Specifically, the tube 80 is held by the first support surface 18a of the first holding portion 15, the second support surface 18b of the second holding portion 16, and the compression member 21a of the cover arm 2.

### (Valve Closed State)

Here, the pinch valve 100 shifts to the closed state.

Fig. 8(b) illustrates a state in which the pinch valve 100 squeezes the tube 80, that is, the pinch valve 100 in the closed state.

Specifically, the press portion 50 moves toward the compression member 21a in the direction along the first axis L1. Then, the tube 80 is squeezed between the press member 5a and the compression member 21a.

Here, in Fig. 8(b), the cover arm 2 is subjected to a heavy load in the direction along the first axis L1 in the closed state. The pinch valve according to the present invention is configured such that surfaces thereof can be subjected to the load in pressure receiving areas D so as to disperse the load.

In the present embodiment, the contact surfaces 22 according to the present invention are formed by the first contact surface 22a and the second contact surface 22b. In addition, the stopper surfaces 19 according to the present invention are formed by the first stopper surface 19a and the second stopper surface 19b.

Specifically, the tip end portion 21g of the cover arm 2 has the second contact surface 22b formed so as to face the one end side in the direction along the first axis L1. In addition, the second frame 14 has the second stopper surface 19b formed so as to face the other end B side in the direction along the first axis L1.

With this configuration, the surface contact between the second stopper surface 19b and the second contact surface 22b in the pressure receiving area D in the second frame 14 enables the load generated by the press portion 50 to be dispersed and received by these surfaces.

Furthermore, the base end portion 21f of the cover arm 2 in the present embodiment has the first contact surface 22a formed so as to face the one end side in the direction along the first axis L1. In addition, the first frame 13 has the first stopper surface 19a formed so as to face the other end B side in the direction along the first axis L1. With this configuration, the load is received by the two pressure receiving areas D in the first frame 13 and the second frame 14 so as to be further dispersed.

Needless to say, a clearance is set between the lock pin 26 and the engagement recess 14d such that the lock pin 26 and the engagement recess 14d do not come into contact with each other even when being subjected to a load.

With this configuration, the pressing force generated by the press portion 50 in the direction along the first axis L1 is received by the surfaces in the pressure receiving areas D of the holder 10. As a result, such a load is dispersed, thus enabling, for example, the lock pin to be inhibited from being damaged.

In the present embodiment, the surface contact is used. However, for example, line contact or point contact achieves a similar effect.

### (Removal of Tube)

First, the operation lever 25 of the lock mechanism 23 slides against the urging spring 28 according to the instruction displayed on the cover arm 2. Thus, the engagement between the lock pin 26 and the engagement recess 14d is released, and the cover arm 2 is pivoted to the position illustrated in Fig. 7 by the urging force of the torsion spring 4. Then, the tube 80 can be removed from the pinch valve 100 by front access.

When the cover arm 2 is not pivoted by the urging force of the torsion spring 4 alone due to the diameter of the tube 80 being large or when the cover arm 2 does not include the torsion spring 4, an operator can remove the tube 80 by pivoting the cover arm 2.

The embodiments of the present invention have been described in detail above. Various design changes can be made without departing from the gist of the present invention. Reference Signs List
- 100: pinch valve
- 10: holder
- 1: holder main body
- 11: holder base
- 11a: connection portion
- 11b: through hole
- 12: holding groove
- 13: first frame
- 13a: first recess
- 13c: first slide groove
- 13d: pivot axis hole
- 13e: step portion
- 14: second frame
- 14a: second recess
- 14c: second slide groove
- 14d: engagement recess
- 14e: arm guide inclined surface
- 14f: lock guide inclined surface
- 15: first holding member
- 15a: first fixing screw
- 16: second holding member
- 16a: second fixing screw
- 17: opening
- 18: support surface
- 18a: first support surface
- 18b: second support surface
- 19: stopper surface
- 19a: first stopper surface
- 19b: second stopper surface
- 2: cover arm
- 21: arm main body
- 21a: compression member
- 21b: arm follow inclined surface
- 21e: pivot axis hole
- 21f: base end portion
- 21g: tip end portion
- 21h: torsion spring receiving portion
- 22: contact surface
- 22a: first contact surface
- 22b: second contact surface
- 23: lock mechanism
- 24: lock base
- 24a: slide hole
- 24b: slit
- 25: operation lever
- 25a: grip portion
- 25b: display board
- 26: lock pin
- 26a: lock follow inclined surface
- 26b: elongated hole
- 26c: fixing pin
- 26d: fixing hole
- 26e: connection wall
- 26f: urging spring receiving portion
- 27: lightening hole
- 28: urging spring
- 3: pivot axis
- 4: torsion spring
- 50: press portion
- 5a: press member
- 5b: connection recess
- 5c: slide projection
- 5d: fixing clip
- 70 (70a, 70b): drive unit
- 7: air cylinder
- 71: piston
- 71a: detection magnet
- 71b: gasket
- 71c: piston ring
- 72: piston rod
- 72a: connection projection
- 73: cylinder main body
- 73a: connection hole
- 73b: rod-side partition wall
- 73c: end-side partition wall
- 73d: first air inlet and outlet hole
- 73e: second air inlet and outlet hole
- 74: press spring
- 74a: auxiliary press spring
- 80: tube
- A: one end
- B: other end
- D: pressure receiving area
- L1: first axis
- L2: second axis
- L3: pivot axis
- Po: open position
- Pc: closed position

## Claims

1. A pinch valve comprising:
a holder having a holding groove for holding a tube made of an elastic material;
a press portion that is supported so as to be able to reciprocate in a direction along a first axis in the holding groove and that is configured to squeeze the tube held in the holding groove; and
a drive unit configured to reciprocate the press portion, wherein
the holding groove extends in a direction along a second axis orthogonal to the first axis and has an opening that is open along the first axis at one end in the direction along the first axis and through which the tube can be inserted into and removed from the holding groove, and
the holder includes a cover arm configured to open and close the opening.

2. The pinch valve according to Claim 1, wherein
the holder includes a first frame and a second frame that extend along the first axis with the first axis interposed therebetween, the holding groove being formed between a pair of the frames, and
the first frame has a pivot axis parallel to the first axis, the cover arm being supported so as to be pivotable on the first frame around the pivot axis between a closed position in which the cover arm closes the opening and an open position in which the cover arm opens the opening.

3. The pinch valve according to Claim 2, wherein the cover arm in the closed position is detachably locked into the second frame in a pivoting direction around the pivot axis.

4. The pinch valve according to Claim 3, wherein the cover arm includes a lock pin provided so as to be able to advance and retreat, the second frame having an engagement recess with which the advanced lock pin is engaged, the lock pin and the engagement recess being configured to be engageable with each other in the closed position.

5. The pinch valve according to Claim 4, wherein the lock pin is provided so as to be able to advance toward a tip end side of the cover arm and retreat and is always urged by an urging spring in an advance direction of the lock pin.

6. The pinch valve according to Claim 5, wherein a tip end portion of the lock pin has a follow inclined surface, the lock pin being configured to retreat against an urging force of the urging spring by the follow inclined surface coming into contact with the second frame when the cover arm pivots to the closed position.

7. The pinch valve according to Claim 6, wherein the cover arm includes an operation lever for releasing engagement between the lock pin and the engagement recess by retreating the lock pin against the urging force of the urging spring.

8. The pinch valve according to Claim 2, wherein
a tip end portion of the cover arm has a contact surface formed so as to face one end side in the direction along the first axis, the second frame having a stopper surface formed so as to face another end side in the direction along the first axis, and
in the closed position of the cover arm, the contact surface and the stopper surface are disposed so as to face each other.

9. The pinch valve according to Claim 8, wherein the first frame further has a stopper surface formed so as to face the other end side in the direction along the first axis.

10. The pinch valve according to Claim 1, wherein the holding groove further has, at respective end portions thereof in the direction along the second axis, arc-shaped support surfaces for coming into contact with and supporting an outer peripheral surface of the tube.

11. The pinch valve according to Claim 1, wherein
the drive unit is an air cylinder joined to the other end side of the holder, the air cylinder including a hollow cylinder main body, and a piston configured to reciprocate in the direction along the first axis in the cylinder main body, and
the press portion is connected to the one end side of the piston.

12. The pinch valve according to Claim 11, wherein the air cylinder includes a press spring configured to always urge the piston toward one of the one end side and the other end side in the direction along the first axis.
